# EUROPEAN PATENT SPECIFICATION

(11) **EP 1 938 851 B1**
(45) Date of publication and mention of the grant of the patent: **24.10.2018**
(21) Application number: 07024175.7
(22) Date of filing: 13.12.2007
(51) Int. Cl.: A61M 5/168, A61M 39/08, A61M 5/142

(54) **Administration set with two keys**
Verabreichungsset mit zwei Schlüsseln
Ensemble d'administration à deux clés

(30) Priority: 25.12.2006 IL 18030506
(43) Date of publication of application: 02.07.2008
(73) Proprietor: Caesarea Medical Electronics Ltd., 38900 Caesarea (IL)
(72) Inventor: Barak, Swi, Caesarea 38900 (IL)
(74) Representative: Harrison IP Limited

(56) References cited:
- EP-A- 1 247 538
- WO-A-2006/083933
- US-A- 4 798 590
- US-A1- 2005 277 890

## Description

### Field and background of the invention

This invention relates to liquid administration and more specially, it is concerned with a liquid flow set useful for administration of liquids to a patient through a flexible tube.

Systems for administration of liquids to a patient are widely known. However, a variety of different pumps are available for propelling liquid to a patient, which may differ, among others, by construction and safety of use.

Flow sets for use with a liquid pump must be attentively designed for safe use. A dedicated pumping tube-segment of the flow set must be installed in the pump and the tube-segment must be installed in the right place, and held taut, straight and stretched only up to a determined value. The flow set must be full of liquid before using it and should remain full as long as it is in use.

Moreover, the tube-segment must be replaced by a new pumping tube-segment when it loses its flexibility.

US 4,798,590 discloses a flow set comprising a drip chamber, a flexible plastic line, having a roller clamp, an inlet connector coupled to at the end to a pump chamber, wherein the other end of the pump chamber is coupled to an outlet connector. The outlet connector is coupled through a line having a roller clamp and a Y-site therein to a luer.

The present invention is concerned with a liquid flow set that includes a number of safety features forming a unique compatibility between a flow set and the liquid pump therefor and ensuring a correct installation of the pumping tube-segment.

### Summary of the invention

According to the present invention, there is provided a flow set as hereinafter set forth in Claim 1 of the appended claims.

Preferred features of the invention are disclosed in the dependent claims.

### Brief description of the figures

The invention is herein described, by a way of example only, with reference to the accompanying drawing. With reference now to the specific detail in the drawings, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawing making it apparent to those skilled in the art how the several forms of the invention may be embodied in practice.

In the figures:
Figure 1 illustrates a flow set with a spike (11), a first administration tube-segment (12a), a pumping tube-segment (12b), a stretching-key (13), an identifying-key (14), a second administration tube-segment (12c) and an anti-free-flow valve (15),
Figure 2 illustrates the installation of a pumping tube segment (12b) in a liquid pump (21),
Figure 3 illustrates a cross-section of the identifying-key (14), and
Figure 4 illustrates a cross-section of the stretching-key (13).

### Description the preferred embodiment

The flow set of the present invention has a number of features that ensure the use of only a specific flow set with a compatible liquid pump. There is an identifying-key that prevents the insertion of a wrong flow set and enables the liquid pump to determine the presence of the compatible administration set. In addition, the flow set of the present invention has a number of features that ensure the use of the flow set in a way that prevents over stretching of the pumping tube-segment.

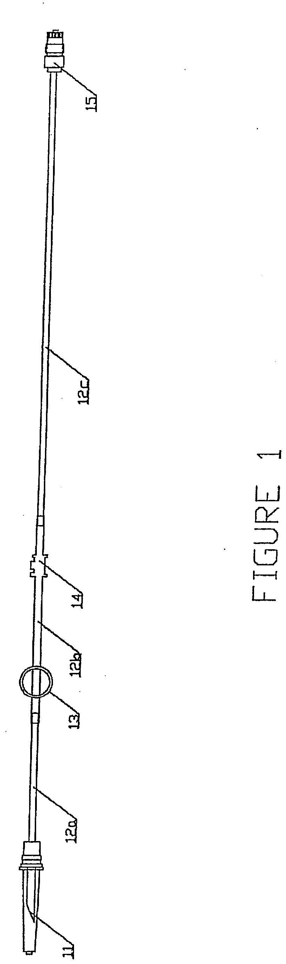

Referring now to Figure 1, that illustrates a flow set with a spike 11, a first administration tube-segment 12a, a pumping tube-segment 12b, a second administration tube 12c and an anti-free-flow valve 15. An identifying-key 14 is installed near one of the pumping tube-segment's ends. Following installation of the identifying-key 14 in a liquid pump 21 the stretching-key 13 is used to install the pumping tube-segment 12b in the liquid pump 21 in a correct position, and stretched up to a determined degree.

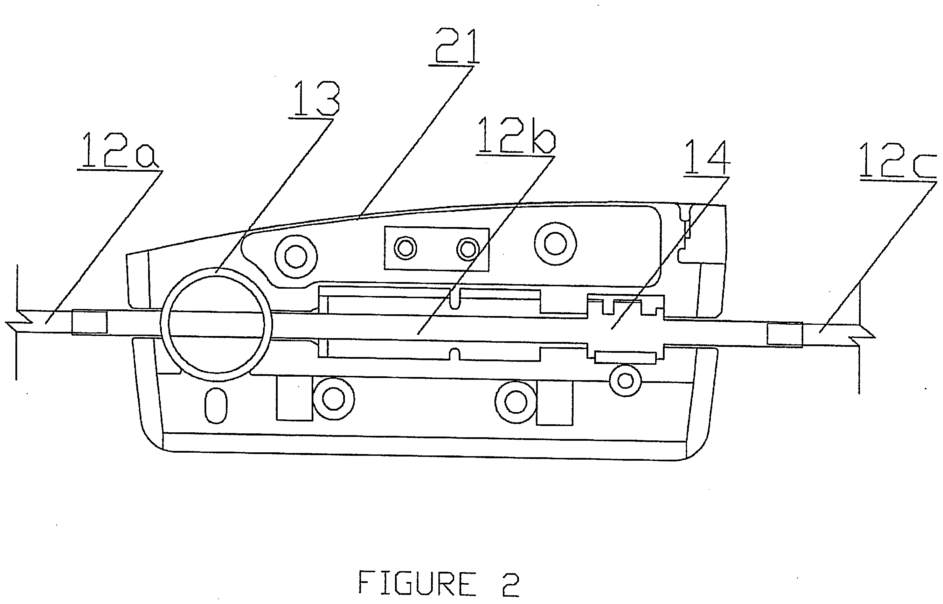

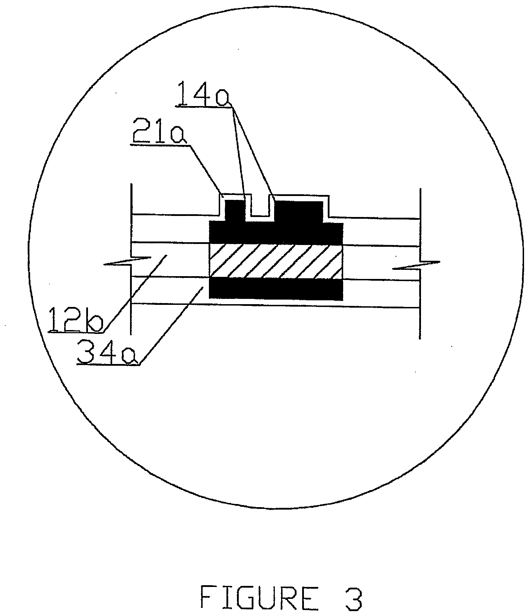

Figure 2 illustrates the way to install a pumping tube-segment 12b in a liquid pump 21. An identifying-key 14 is disposed on the pumping tube-segment 12b. The identifying-key 14 includes a collar that is clamped onto the tube-segment 12b and has a number of teeth 14a, shown in more detail in Figure 3. Each tooth has a specific width and specific location, creating a code that allows the inserting of the tube-segment 12b only to a specific liquid pump 21 that has a set of niches 21a that are arranged in accord with the same unique structural code.

The identifying-key 14 has also a pressure-plate 34a. This pressure-plate 34a is pressed by a door (not shown) of the liquid pump 21 and presses the pumping tube-segment 12b against a pressure-sensor (not shown) that is installed in the liquid pump 21 behind the identifying-key 14. The identifying-key 14 prevents the liquid pump 21 from starting operation unless the pressure-sensor measures a determined pressure. The stretching-key is designed to be installed in a recess 21b of the liquid pump 21. The determined distance between the identifying-key 14 and the stretching-key 13 corresponds to the respective distance between the identifying-key niches 21a and the stretching-key recess 21b in the liquid pump 21, shown in more details in Figure 3. When both the identifying-key and the stretching-key are installed in the liquid pump 21, the determined distance between their locations on the pumping tube-segment 12b ensures that the pumping tube-segment 12b is tightened and held straight and stretched up to a determined degree.

Figure 3 illustrates a cross-section of an identifying-key 14. The identifying-key 14 is clamped onto the tube-segment 12b near one of its ends. The identifying-key 14 includes a set of teeth 14a to enable the insertion of the flow set only into a compatible liquid pump and presses the pumping tube-segment 12b against a pressure-sensor (not shown) of the liquid pump enabling the pressure-measuring.

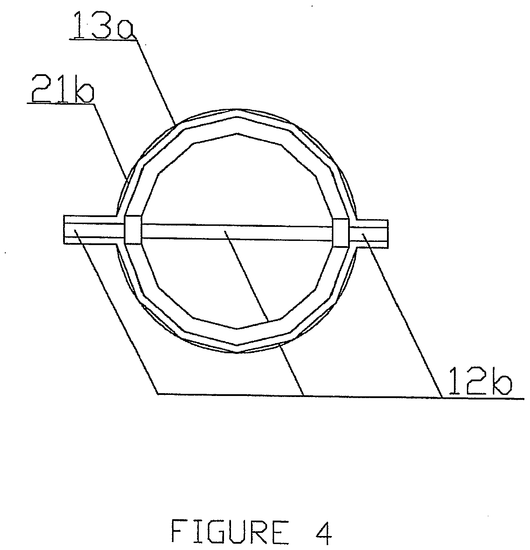

Figure 4 illustrates a cross-section of a stretching-key 13. The stretching-key 13 includes a collar clamped onto the pumping tube-segment 12b near its other end. The stretching-key 13 may be ring-shaped in order to enable an air sensor (not shown) to detect air in the tube-segment part located within the stretching-key centre, ensuring that the pumping tube-segment is placed in front of the air sensor for optimal detection of air in the pumping tube-segment.

Although the invention has been described in conjunction with specific embodiments thereof, it is evident that many alternatives, modifications and variations will be apparent to those skilled in the art, falling within the scope of the invention as defined by the appended claims.

## Claims

1. A disposable flow set (10) comprising:
(a) a drip chamber or a spike (11);
(b) a first administration tube-segment (12a) for administering liquid from said drip chamber or spike (11) having a first end connected to said drip chamber or spike (11);
(c) a pumping tube-segment (12b) having a first end connected to a second end of said first administration tube-segment (12a); said pumping tube-segment (12b) of said flow set (10) being suitable for installation in a liquid pump (21);
(d) a second administration tube-segment (12c) having a first end connected to a second end of said pumping tube-segment (12b);
(e) an anti-free-flow valve (15) at a second end of said second administration tube-segment (12c); and
(f) an identifying-key (14) connected to said pumping tube-segment (12b), said identifying-key (14) being clamped on said pumping tube-segment and including:
(i) a number of teeth (14a) with a unique combination of location and width, said teeth (14a) being insertable into compatible niches (21a) in a specific liquid pump (21) so as to prevent insertion of said flow set to a non-compatible liquid pump; and
(ii) a pressing-plate (34a) to be pressed by a door of the liquid pump (21) in order to allow said pumping tube-segment (12b) to be pressed against a pressure-sensor of said liquid pump when the door of said liquid pump is closed to enable said liquid pump to use the sensed pressure to verify the presence of said flow set;
said disposable flow set (10) being **characterized by**:
(g) a stretching-key (13) connected to said pumping tube-segment (12b); said stretching-key (13) being clamped on the pumping tube-segment (12b) at a distance from the identifying-key (14) and having a unique pattern to be inserted into a compatible recess in a specific liquid pump (21a) to prevent insertion of the flow set in a non-compatible liquid pump, and maintain the pumping tube-segment (12b) under tension, straight, and stretched to a determined degree when inserted into the liquid pump (21a).

2. A disposable flow set of claim 1, wherein the distance between said identifying-key (14) and said stretching-key (13) ranges from about 45 millimeters to about 47 millimeters.

3. A disposable flow set of claim 1, wherein said stretching-key (13) is ring-shaped, in order to allow detection of air in the pumping tube segment (12b) by a liquid pump air detector through the hole in the stretching key (13).

4. A disposable flow set of claim 1, wherein the stretching-key is sized and shaped such as to ensure that, following installation of the identifying key 14, the stretching key is correctly positioned in the liquid pump (21) and stretched to a predetermined degree.

5. A disposable flow set of claim 1 wherein the stretching-key is operative to centre the pumping tube-segment in front of an air sensor detector of the liquid pump (21) to assure optimal air detection in the pumping tube-segment.

## Patentansprüche

1. Einweg-Strömungsset (10), das Folgendes umfasst:
(a) eine Tropfkammer oder einen Dorn (11);
(b) ein erstes Verabreichungsrohrsegment (12a) zum Verabreichen von Flüssigkeit aus der Tropfkammer oder dem Dorn (11), das ein erstes Ende aufweist, das mit der Tropfkammer oder dem Dorn (11) verbunden ist;
(c) ein Pumprohrsegment (12b), das ein erstes Ende aufweist, das mit einem zweiten Ende des ersten Verabreichungsrohrsegments (12a) verbunden ist; wobei das Pumprohrsegment (12b) des Strömungssets (10) dazu geeignet ist, in einer Flüssigkeitspumpe (21) eingebaut zu werden;
(d) ein zweites Verabreichungsrohrsegment (12c), das ein erstes Ende aufweist, das mit einem zweiten Ende des Pumprohrsegments (12b) verbunden ist;
(e) ein Ventil zur Verhinderung freier Strömung (15) an einem zweiten Ende des zweiten Verabreichungsrohrsegments (12c); und
(f) einen Identifizierungsschlüssel (14), der mit dem Pumprohrsegment (12b) verbunden ist, wobei der Identifizierungsschlüssel (14) auf das Pumprohrsegment geklemmt ist und Folgendes beinhaltet:
(i) eine Anzahl von Zähnen (14a) mit einer einzigartigen Kombination von Standort und Breite, wobei die Zähne (14a) in passende Nischen (21a) in einer konkreten Flüssigkeitspumpe (21) einführbar sind, um Einführen des Strömungssets in eine nicht passende Flüssigkeitspumpe zu verhindern; und
(ii) eine Druckplatte (34a), die durch eine Tür der Flüssigkeitspumpe (21) gedrückt werden soll, um dem Pumprohrsegment (12b) zu ermöglichen, gegen einen Drucksensor der Flüssigkeitspumpe gedrückt zu werden, wenn die Tür der Flüssigkeitspumpe geschlossen wird, um der Flüssigkeitspumpe zu ermöglichen, den erfassten Druck zu verwenden, um die Anwesenheit des Strömungssets zu verifizieren;
das Einweg-Strömungsset (10) durch Folgendes gekennzeichnet ist:
(g) einen Streckungsschlüssel (13), der mit dem Pumprohrsegment (12b) verbunden ist; wobei der Streckungsschlüssel (13) mit einer Entfernung von dem Identifizierungsschlüssel (14) auf das Pumprohrsegment (12b) geklemmt ist und ein einzigartiges Muster aufweist, das in eine passende Vertiefung in einer konkreten Flüssigkeitspumpe (21a) eingeführt werden soll, um Einführen des Strömungssatzes in eine nicht passende Flüssigkeitspumpe zu verhindern und das Pumprohrsegment (12b) unter Spannung, gerade und mit einem bestimmten Grad gestreckt zu halten, wenn es in die Flüssigkeitspumpe (21a) eingeführt wird.

2. Einweg-Strömungsset nach Anspruch 1, wobei die Entfernung zwischen dem Identifizierungsschlüssel (14) und dem Streckungsschlüssel (13) von etwa 45 Millimeter zu etwa 47 Millimeter reicht.

3. Einweg-Strömungsset nach Anspruch 1, wobei der Streckungsschlüssel (13) ringförmig ist, um Detektion von Luft in dem Pumprohrsegment (12b) durch einen Luftdetektor der Flüssigkeitspumpe durch das Loch in dem Streckungsschlüssel (13) zu ermöglichen.

4. Einweg-Strömungsset nach Anspruch 1, wobei die Streckungsgröße so bemessen und geformt ist, um sicherzustellen, dass nach dem Einbauen des Identifizierungsschlüssels 14 der Streckungsschlüssel richtig in der Flüssigkeitspumpe (21) positioniert ist und bis zu einem vorbestimmten Grad gestreckt ist.

5. Einweg-Strömungsset nach Anspruch 1, wobei der Streckungsschlüssel dazu betriebswirksam ist, das Pumprohrsegment vor einem Luftsensordetektor der Flüssigkeitspumpe (21) zu zentrieren, um optimale Luftdetektion in dem Pumprohrsegment sicherzustellen.

## Revendications

1. Dispositif d'écoulement à usage unique (10) comprenant :
(a) une chambre compte-gouttes ou une pointe (11) ;
(b) un premier segment de tube d'administration (12a) pour l'administration de liquide depuis la chambre compte-gouttes ou la pointe (11) possédant un premier bout raccordé audit compte-gouttes ou pointe (11) ;
(c) un segment de tube de pompage (12b) possédant un premier bout raccordé à un deuxième bout dudit premier segment de tube d'administration (12a), ledit segment de tube de pompage (12b) dudit dispositif d'écoulement (10) convenant pour l'installation dans une pompe de liquide (21);
(d) un deuxième segment de tube d'administration (12c) possédant un premier bout raccordé à un deuxième bout dudit segment de tube pompage (12b) ;
(e) une soupape anti-écoulement libre (15) à un deuxième bout dudit deuxième segment de tube d'administration (12c) ; et
(f) une clé d'identification (14) connectée audit segment de tube pompage (12b), ladite une clé d'identification (14) étant fixée sur ledit segment de tube pompage, et comprenant :
(i) un nombre de dents (14a) avec une combinaison unique d'emplacement et de largeur, lesdites dents (14a) pouvant être introduites dans des créneaux compatibles (21a) dans une pompe de liquide spécifique (21) de façon à empêcher l'insertion dudit dispositif d'écoulement dans une pompe de liquide non spécifique ; et
(ii) une plaque de pression (34a) devant être pressée par une porte de la pompe de liquide (21) afin de permettre audit segment de tube pompage (12b) d'être pressé contre un capteur de pression de ladite pompe de liquide lorsque la pompe de ladite pompe de liquide est fermée afin de permettre à ladite pompe de liquide d'utiliser le capteur de pression pour vérifier la présence dudit dispositif d'écoulement ;
ledit dispositif d'écoulement à usage unique (10) étant **caractérisé par** :
(g) une clé d'étirage (13) raccordée audit segment de tube pompage (12b) ; ladite clé d'étirage (13) étant fixée sur le segment de tube pompage (12b) à une distance de la clé d'identification (14), et possédant une configuration unique pour être insérée dans un évidement compatible dans une pompe de liquide spécifique (21a) de façon à empêcher l'insertion du dispositif d'écoulement dans une pompe de liquide non compatible, et à maintenir le segment de tube pompage (12b) sous tension, droit, et étiré jusqu'à un degré déterminé lorsqu'il est inséré dans la pompe de liquide (21a).

2. Dispositif d'écoulement à usage unique selon la revendication 1, la distance entre ladite clé d'identification (14) et ladite clé d'étirage (13) allant d'environ 45 millimètres à environ 47 millimètres.

3. Dispositif d'écoulement à usage unique selon la revendication 1, ladite clé d'étirage (13) étant annulaire afin de permettre la détection d'air dans le segment de tube pompage (12b) par un détecteur d'air de la pompe de liquide à travers l'orifice dans la clé d'étirage (13).

4. Dispositif d'écoulement à usage unique selon la revendication 1, les dimensions et la forme de la clé d'étirage permettant d'assurer qu'à la suite de l'installation de la clé d'identification (14), la clé d'étirage est positionnée correctement dans la pompe de liquide (21) et étirée jusqu'à un degré déterminé.

5. Dispositif d'écoulement à usage unique selon la revendication 1, la clé d'étirage fonctionnant pour centrer le segment de tube pompage devant le détecteur d'air de la pompe de liquide (21) afin d'assurer une détection d'air optimale dans le segment de tube pompage.
